Europäisches Patentamt

European Patent Office

Office européen des brevets

(19)

(11) Veröffentlichungsnummer : **0 070 543**
**B1**

(12) **EUROPÄISCHE PATENTSCHRIFT**

(45) Veröffentlichungstag der Patentschrift :
**09.10.85**

(21) Anmeldenummer : **82106437.5**

(22) Anmeldetag : **16.07.82**

(51) Int. Cl.⁴ : **A 61 L 25/00, A 61 F 2/28,
A 61 F 2/30, A 61 F 2/32,
A 61 K 49/04**

(54) **Pulvermischung für chirurgische Zwecke.**

(30) Priorität : **22.07.81 DE 3128923**

(43) Veröffentlichungstag der Anmeldung :
**26.01.83 Patentblatt 83/04**

(45) Bekanntmachung des Hinweises auf die Patenterteilung : **09.10.85 Patentblatt 85/41**

(84) Benannte Vertragsstaaten :
**AT BE CH DE FR GB IT LI NL SE**

(56) Entgegenhaltungen :
**DE-A- 2 715 532
DE-B- 2 552 070
GB-A- 2 069 517**

(73) Patentinhaber : **Beiersdorf Aktiengesellschaft
Unnastrasse 48
D-2000 Hamburg 20 (DE)**

(72) Erfinder : **Pietsch, Hanns, Dr.
Mittelweg 25
D-2000 Hamburg 13 (DE)**
Erfinder : **Eckloff, Willi
Bargteheider Strasse 145a
D-2000 Hamburg 73 (DE)**
Erfinder : **Hohmann, Volker
Theodor Fontane Strasse 7a
D-2000 Norderstedt (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Pulvermischung für aushärtende chirurgische Massen auf Basis Polymethylmethacrylat, sogenannte Knochenzemente.

Derartige Massen werden vorzugsweise zur Implantation von Gelenkprothesen, darunter am häufigsten zur Implantation von Hüftgelenkprothesen verwendet. Weitere Indikationen sind die Verbundosteosynthesen bei Spontanfrakturen und Frakturen osteoporotischer Knochen. Noch andere Indikationen, wie Ersatz von Skelettanteilen und Fusionen der Wirbelsäule, wurden ausführlicher in dem Buch O. Oest, K. Müller, W. Hupfauer ; « Die Knochenzemente », F. Enke Verlag, Stuttgart 1975, S. 14-26 beschrieben.

Diese Massen sind Zweikomponentensysteme, die aus einer Pulvermischung und einer Flüssigkeit bestehen. Die Pulvermischung besteht aus Polymerpulver, vorzugsweise Polymethylmethacrylatpulver oder pulverförmigen Mischpolymerisaten mit Methylmethacrylateinheiten, Röntgenkontrastmitteln wie Bariumsulfat oder Zirkondioxid, und dem Polymerisationsinitiator, einem organischen Peroxid, vorzugsweise Dibenzoylperoxid. Die flüssige Phase besteht hauptsächlich aus Methylmethacrylat und/oder anderen Acryl- oder Methacrylsäureestern, und einem Beschleuniger, üblicherweise N,N-Dimethyl-p-toluidin. Solche Knochenzemente beschreibt die DE-AS-2 552 070.

Knochenzemente verbleiben nach der Implantation dauernd im menschlichen Körper. Sie werden in vielen Ländern Arzneimitteln gleichgesetzt und unterliegen der Anmeldepflicht bei den staatlichen Gesundheitsämtern. Dementsprechend werden an die Reinheit der Rohstoffe entsprechend hohe Anforderungen gestellt. Dies gilt für alle Bestandteile : Polymerisatpulver, Röntgenkontrastmittel und Polymerisationsinitiator.

Der in Knochenzementen üblicherweise benutzte Polymerisationsinitiator ist Benzoylperoxid. Benzoylperoxid ist feuergefährlich und explosiv. Es darf aus Sicherheitsgründen nicht in reiner Form in den Handel kommen oder transportiert werden.

Aus diesem Grund werden handelsübliche Benzoylperoxidtypen « phlegmatisiert », d. h. sie werden mit nicht explosiven, nicht feuergefährlichen Beimengungen versehen. Solche Beimengungen sind beispielsweise Wasser oder Weichmacher wie Dibutylphthalat Wegen der beschriebenen Reinheitsforderungen kommt ein mit Weichmacher verschnittener Polymerisationsinitiator als Bestandteil für einen Knochenzement nicht in Frage. Jedoch wäre es denkbar, wasserfeuchtes Benzoylperoxid zu verwenden.

Derartige wasserfeuchte Benzoylperoxide sind wegen ihres Wassergehaltes etwas klebrig, sie ballen sich zu lockeren Aggregaten zusammen und sind schlecht rieselfähig. Dies bedeutet, daß damit hergestellte Pulvermischungen eine inhomogene Peroxidverteilung aufweisen : einzelne Teile einer Charge enthalten unterschiedliche Peroxidmengen und innerhalb einer Packungseinheit gibt es « Peroxidnester ».

Beide Effekte sind unerwünscht. Der erste Effekt bedeutet, daß eine einheitliche Dosierung des Peroxids nicht gegeben ist, da einzelne Packungen einer Herstellungscharge unterschiedliche Peroxidgehalte aufweisen. Der zweite Effekt ist ebenso unangenehm und unerwünscht, er führt zu Beeinträchtigungen im ausgehärteten Knochenzement.

Obwohl beim Anrühren eines Knochenzementes, beim Vermischen der Pulverphase mit der flüssigen Phase ein Mischvorgang stattfindet, reicht dieser sehr häufig nicht aus, um Inhomogenitäten der Pulvermischung auszugleichen, denn je nach Rezeptur geschieht dieser Mischvorgang innerhalb von 2 bis 5 Minuten. Man muß im Gegenteil damit rechnen, daß sich in ungünstigen Fällen die Inhomogenitäten nicht ausgleichen, sondern kumulieren.

Im ausgehärteten Knochenzement können dann folgende negativen Effekte auftreten : inhomogene Festigkeitsverteilung, inhomogene Wärmeverteilung beim Aushärten, inhomogene Restmonomerverteilung, sowie inhomogene Restperoxidverteilung, die letzten Endes alle die Lockerung der Prothese begünstigen können.

Die Aufgabe der Erfindung bestand darin, die aufgezeigten Nachteile zu vermeiden, insbesondere eine homogene Verteilung des Polymerisationsinitiators in der Pulvermischung zu gewährleisten, ohne daß Fremdstoffe in die Mischung zusätzlich eingebracht werden müssen. Gleichzeit sollte die sichere Handhabung des Initiators gewährleistet sein und die entsprechenden Sicherheitsauflagen berücksichtigt werden.

Diese Aufgabe wird erfindungsgemäß dadurch gelöst, daß bei Zweikomponentensystemen aus Pulvermischung und Flüssigkeit die Pulvermischung wesentlich verbessert wird, nämlich dadurch, daß der Polymerisationsinitiator nicht allein, sondern als ein Überzug auf einem der beiden anderen für derartige chirurgische Massen charakteristischen Bestandteile verwendet wird. Die erfindungsgemäßen Pulvermischungen sind also dadurch gekennzeichnet, daß Polymerpulver- bzw. Röntgenkontrastmittelpartikel als Träger dienen, die vom Initiator umhüllt werden. In einer Ausführungsform dient das Röntgenkontrastmittel Zirkondioxid als Träger für einen Benzoylperoxidüberzug. Der Anteil des Peroxids kann dabei zwischen 1 bis 50 Gew.-% liegen, vorzugsweise bei 2 bis 25 Gew.-%, ganz besonders bei 5-20 Gew.-%. Der Anteil Röntgenkontrastmittel beträgt dementsprechend 99-50 Gew.-%, vorzugsweise 98-75 Gew.-%. Mit diesen Kombinationsprodukten lassen sich z. B. die Pulvermischungen gemäß der DE-AS-2 552 070 in den dort angegebenen Mengenverhältnissen herstellen, die wie dort angegeben, mit einer homogenen Wasseremulsion umgesetzt werden können.

**0 070 543**

Die Herstellung derartiger Kombinationsprodukte kann in an sich bekannter Weise erfolgen, z. B. derart, daß das zu überziehende Material unter Bewegung getrocknet wird, während das Überzugsmaterial als Lösung aufgebracht wird. Das Lösungsmittel für das Überzugsmaterial sollte dabei das zu überziehende Material nicht lösen. Beispiele für derartige Verfahren sind die Wirbelschicht-Trocknung oder die Herstellung von Dragees. Geeignete Lösungsmittel sind z. B. Äthanol oder Äther bei der Beschichtung von Polymethylmethacrylat mit Benzoylperoxid. Bei der Beschichtung von Röntgenkontrastmittel mit Benzoylperoxid sind auch chlorierte Kohlenwasserstoffe, wie Methylenchlorid, Trichlormethan oder Tetrachlormethan geeignet.

Die mit Hilfe dieser Kombinationsprodukte herzustellenden erfindungsgemäßen Pulvermischungen können je nach Art des Kombinationsproduktes insbesondere

a) entweder aus dem Kombinationsprodukt und Polymethylmethacrylat bestehen, wenn das Kombinationsprodukt aus Röntgenkontrastmittel und Initiator besteht, oder

b) aus dem Kombinationsprodukt und Röntgenkontrastmittel, wenn das Kombinationsprodukt aus Polymerpulver und Initiator besteht.

Die Verwendung eines derartigen Kombinationsproduktes ergibt homogene Pulvermischungen, wobei der stationäre Zustand früher erreicht wird als bei der Verwendung von mit Wasser phlegmatisiertem Benzoylperoxid. Überraschend wurde zusätzlich gefunden, daß bei Verwendung eines Kombinationsproduktes mit Polymerisationsinitiator auf der Oberfläche von Pulverpartikeln die effektive Oberfläche des Initiators größer ist als bei der Verwendung der bisher üblichen kristallinen Typen, die zudem noch teilweise durch Wasser verklumpt waren. Es ist somit möglich, bei gleichem Peroxidgehalt in der Pulvermischung die Aushärtereaktion beim erfindungsgemäßen Verfahren zu beschleunigen oder bei gleicher Aushärtegeschwindigkeit den Peroxidgehalt zu verringern. Da im ausgehärteten Knochenzement Restperoxid toxisch ist, ist dieses eine Verbesserung der Qualität des Knochenzements.

Es zeigte sich auch, daß durch die Beschichtung mit Initiator der Durchmesser der Partikel maximal um ca. 20 % steigt. Wenn beispielsweise die Komponente mit dem kleinsten mittleren Partikeldurchmesser als Träger für Initiator benutzt wird, wird der maximale Partikeldurchmesser nicht überschritten.

Es zeigte sich, daß bei Verwendung maximaler Beschichtungen von 50 % der maximale Partikeldurchmesser von 200 Mikrometer nicht überschritten wurde.

Beispiel 1

Vergleich mit dem Stand der Technik gemäß DE-AS-2 552 070.

Es wurden drei Pulvermischungen zu je 1 kg durch Vermischen folgender Bestandteile hergestellt :

882,5 g Polymethylmethacrylatpulver
100,0 g Zirkondioxidpulver
 17,5 g Dibenzoylperoxid, 80 %ig wasserfeucht

Je 1 kg wurde 30 Minuten, 60 Minuten und 120 Minuten in einem Doppelkegel-Pulvermischer vermischt, sodann je in Portionen von 50 g aufgeteilt und dann von jeder Portion zwei Peroxidbestimmungen durch jodometrische Titration durchgeführt.

Die Ergebnisse sind in Tabelle 1 zusammengestellt.

Die Einzelwerte wurden statistisch verarbeitet und s die Standardabweichung, $\bar{x}$ das arithmetische Mittel, V der Variationskoeffizient, sowie $V_r$ der relative Variationskoeffizient bestimmt.

$$\bar{x} = \frac{\sum x_i}{n} \qquad V = \frac{s}{\bar{x}}$$

$$s^2 = \frac{\sum (x_1 - \bar{x})^2}{n - 1} \qquad V_r \, \% = \frac{\dfrac{s}{\bar{x}}}{\sqrt{n - 1}} \cdot 100$$

nach Lothar Sachs, Angewandte Statistik und Erwin Kreyszig, Statistische Methoden und ihre Anwendung.

Der prozentuale Analysenfehler der Peroxidbestimmung aus allen Werten (Vergleichsversuch und erfindungsgemäßes Beispiel, n = 80) wurde nach der Formel

$$F \, \% = \frac{2 \cdot [x_1 - x_j]}{x_1 + x_j} \cdot 100$$

ermittelt und beträgt 2,84 % relativ.

Ansatz 1 wurde 30′ auf einem Pulvermischer gemischt

3

0 070 543

Ansatz 2 wurde 60' auf einem Pulvermischer gemischt
Ansatz 3 wurde 120' auf einem Pulvermischer gemischt

Jeder Ansatz wurde in 20 Proben zu je 50 g aufgeteilt und von jeder Probe der Peroxidgehalt jodometrisch bestimmt, die Ergebnisse sind in Tabelle 1 aufgelistet. Man erkennt, daß 30' als Mischzeit zu kurz sind, nach 1 Stunde wird ein Ergebnis erzielt, das auch nach 2 Stunden nicht zu verbessern ist. Man erkennt die Inhomogenität der Mischungen an dem relativen Variationskoeffizienten, der von 10,2 % nach 30 Min. auf 1,7 % nach 60 Min. und 120 Minuten abfällt.

Tabelle 1

Peroxidbestimmung im Knochenzemtentpulver

| Nr. | 30' Mischzeit BPO | | | 60' Mischzeit | | | 120' Mischzeit | | |
|---|---|---|---|---|---|---|---|---|---|
| | I | II | Δ | I | II | Δ | I | II | Δ |
| 1 | 1,21 | 1,27 | 0,06 | 1,31 | 1,27 | 0,04 | 1,33 | 1,35 | 0,02 |
| 2 | 1,20 | 1,35 | 0,15 | 1,32 | 1,30 | 0,02 | 1,53 | 1,55 | 0,02 |
| 3 | 1,38 | 1,34 | 0,04 | 1,32 | 1,31 | 0,01 | 1,52 | 1,48 | 0,04 |
| 4 | 1,10 | 1,18 | 0,08 | 1,52 | 1,54 | 0,02 | 1,47 | 1,44 | 0,07 |
| 5 | 1,24 | 1,23 | 0,01 | 1,45 | 1,37 | 0,08 | 1,42 | 1,45 | 0,03 |
| 6 | 1,40 | 1,30 | 0,10 | 1,31 | 1,29 | 0,02 | 1,31 | 1,34 | 0,03 |
| 7 | 1,28 | 1,33 | 0,05 | 1,36 | 1,33 | 0,03 | 1,42 | 1,46 | 0,04 |
| 8 | 1,21 | 1,15 | 0,06 | 1,21 | 1,29 | 0,08 | 1,24 | 1,27 | 0,03 |
| 9 | 1,23 | 1,20 | 0,03 | 1,53 | 1,48 | 0,05 | 1,45 | 1,42 | 0,03 |
| 10 | 1,35 | 1,36 | 0,01 | 1,56 | 1,49 | 0,07 | 1,55 | 1,53 | 0,02 |
| 11 | 1,13 | 1,20 | 0,07 | 1,51 | 1,53 | 0,02 | 1,37 | 1,37 | 0,01 |
| 12 | 1,35 | 1,31 | 0,04 | 1,40 | 1,46 | 0,06 | 1,60 | 1,67 | 0,07 |
| 13 | 1,12 | 1,15 | 0,03 | 1,52 | 1,53 | 0,01 | 1,40 | 1,47 | 0,07 |
| 14 | 1,31 | 1,28 | 0,03 | 1,45 | 1,48 | 0,03 | 1,34 | 1,37 | 0,03 |
| 15 | 1,29 | 1,21 | 0,08 | 1,41 | 1,49 | 0,08 | 1,31 | 1,34 | 0,03 |
| 16 | 1,18 | 1,11 | 0,07 | 1,35 | 1,34 | 0,01 | 1,49 | 1,45 | 0,04 |
| 17 | 1,26 | 1,33 | 0,07 | 1,48 | 1,42 | 0,06 | 1,33 | 1,35 | 0,02 |
| 18 | 1,51 | 1,50 | 0,01 | 1,49 | 1,50 | 0,01 | 1,49 | 1,44 | 0,05 |
| 19 | 4,00 | 4,02 | 0,02 | 1,29 | 1,30 | 0,01 | 1,53 | 1,51 | 0,02 |
| 20 | 1,22 | 1,20 | 0,02 | 1,43 | 1,42 | 0,01 | 1,25 | 1,24 | 0,01 |
| $\bar{x}$ | 1,37 | 1,38 | | 1,41 | 1,407 | | 1,418 | 1,425 | |
| s | 0,6 | 0,3 | | ± 0,098 | ±0,096 | | 0,105 | 0,100 | |
| n | 20 | 20 | | 20 | 20 | | 20 | 20 | |
| v | 0,444 | 0,445 | | 0,070 | 0,068 | | 0,074 | 0,071 | |
| $v_r$ (%) | 10,2 | 10,2 | | 1,6 | 1,6 | | 1,7 | 1,6 | |

Beispiel 2 (erfindungsgemäß)

886 g Polymethylmethacrylatpulver
100 g eines Kombinationsproduktes aus 86 Gew.-% Zircondioxidpulver und 14 Gew.-% Benzoylperoxid
14 g Zirkondioxidpulver

wurden wie im Biespiel 1 eine Stunde im Pulvermischer vermischt und sodann jodometrisch der Peroxidgehalt der Mischung bestimmt:

4

Ergbnisse Tabelle 2

Peroxidgehalt der Probe :

|  | I | II | $\triangle$ |
|---|---|---|---|
| 1 | 1, 40 | 1, 43 | O, O3 |
| 2 | 1, 43 | 1.41 | O, O2 |
| 3 | 1, 35 | 1, 40 | O, O5 |
| 4 | 1, 44 | 1, 44 | O, O5 |
| 5 | 1, 45 | 1, 41 | O, O4 |
| 6 | 1, 35 | 1, 38 | O, O3 |
| 7 | 1, 38 | 1, 35 | O, O3 |
| 8 | 1, 41 | 1, 44 | O, O3 |
| 9 | 1, 41 | 1, 44 | O, O3 |
| 1O | 1, 40 | 1, 38 | O, O2 |
| 11 | 1, 44 | 1, 40 | O, O4 |
| 12 | 1, 40 | 1, 36 | O, O4 |
| 13 | 1, 38 · | 1, 35 | O, O3 |
| 14 | 1, 38 | 1, 39 | O, O1 |
| 15 | 1, 46 | 1, 41 | O, O5 |
| 16 | 1, 35 | 1, 39 | O, O4 |
| 17 | 1, 39 | 1, 45 | O, O6 |
| 18 | 1, 49 | 1, 41 | O, O8 |
| 19 | 1, 38 | 1, 40 | O, O2 |
| 20 | 1, 41 | 1, 46 | O, O5 |
| n | 2O | 2O | |
| $\bar{x}$ | 1, 4O5 | 1, 4O6 | |
| s | O, O38 | O, O33 | |
| v | O, O27 | O, O23 | |
| $V_r$ (%) | O, 6 | O, 5 | |

In der erfindungsgemäßen Mischung erhält man in einer Stunde eine Mischung mit $V_r$ = 0,5, das ist eine dreimal bessere Homogenität, als gemäß Beispiel 1 (Stand der Technik) erzielt wird.

Beispiel 3 (erfindungsgemäß)

Wie im Beispiel 1 beschreiben, jedoch unter Verwendung eines Kombinationsproduktes aus 85 % Polymethylmethacrylat und 15 % Benzoylperoxid :
803,2 g Polymethylmethacrylatpulver
100,0 g Zirkondioxidpulver
93,3 g eines Kombinationsproduktes aus 85 Gew.-% Polymethylmethacrylat und 15 Gew.-% Benzoylperoxid wurden eine Stunde im Pulvermischer vermischt, in einzelne Proben von je 50 g aufgeteilt und von diesen Proben der Peroxidgehalt jodometrisch bestimmt. Der theoretische Peroxidgehalt beträgt 1,404 %.
Es wurden folgende Peroxidgehalte gemessen (Tabelle 3) :

(Siehe Tabelle 3 Seite 6 f.)

# 0 070 543

## Tabelle 3

| Nr. | Peroxidgehalt Messung 1 | Peroxidgehalt Messung 2 | Differenz zwischen Messung 1 u. 2 |
|---|---|---|---|
| 1 | 1,39 | 1,35 | 0,04 |
| 2 | 1,37 | 1,36 | 0,01 |
| 3 | 1,37 | 1,40 | 0,03 |
| 4 | 1,47 | 1,4o | 0,07 |
| 5 | 1,36 | 1,38 | 0,02 |
| 6 | 1,38 | 1,44 | 0,06 |
| 7 | 1,48 | 1,40 | 0,08 |
| 8 | 1,39 | 1,41 | 0,02 |
| 9 | 1,42 | 1,45 | 0,03 |
| 1o | 1,41 | 1,44 | 0,03 |
| 11 | 1,44 | 1,42 | 0,02 |
| 12 | 1,34 | 1,39 | 0,05 |
| 13 | 1,43 | 1,43 | 0,00 |
| 14 | 1,46 | 1,40 | 0,06 |
| 15 | 1,36 | 1,37 | 0,01 |
| 16 | 1,37 | 1,36 | 0,01 |
| 17 | 1,40 | 1,45 | 0,05 |
| 18 | 1,40 | 1,43 | 0,03 |
| 19 | 1,41 | 1,31 | 0,02 |
| 20 | 1,45 | 1,41 | 0,04 |
| $n$ | 20 | 20 | 20 |
| $\bar{x}$ | 1,405 | 1,405 | 0,034 |
| $s$ | 0,034 | 0,032 | |
| $v$ | 0,024 | 0,022 | |
| $V_r$ (%) | 0,55 | 0,505 | |

Auch hier wird eine Mischung mit einer gegenüber dem Stand der Technik dreifach verbesserten Homogenität erhalten ($V_r$ % im Beispiel 3 : 0,05 bzw. 0,505 gegenüber 1,6 im Beispiel 1, das den Stand der Technik repräsentiert).

## Patentansprüche

1. Pulvermischung für chirurgische Zwecke, enthaltend Methylmethacrylat-Homo- und/oder-Mischpolymerisatpulver, Röntgenkontrastmittelpulver und Peroxid-Polymerisationsinitiator, dadurch gekennzeichnet, daß der Peroxid-Polymerisationsinitiator als Überzug auf dem Polymerisatpulver oder dem Röntgenkontrastmittel vorliegt.

2. Pulvermischung nach Anspruch 1, dadurch gekennzeichnet, daß das Röntgenkontrastmittel Zirkondioxidpulver ist, das mit Benzoylperoxid als Initiator überzogen ist.

3. Pulvermischung nach Anspruch 1, dadurch gekennzeichnet, daß das Polymerisatpulver mit Benzoylperoxid überzogen ist.

4. Pulvermischung nach Anspruch 1, dadurch gekennzeichnet, daß der Gehalt an Initiator 1-50 Gew.-%, insbesondere 5-20 Gew.-% beträgt.

5. Pulvermischung nach Anspruch 1, dadurch gekennzeichnet, daß der Gehalt an Röntgenkontrastmittel bis zu 15 Gew.-%, insbesondere 5-10 Gew.-% beträgt.

6

**0 070 543**

6. Pulvermischung nach Anspruch 1, dadurch gekennzeichnet, daß der maximale Partikeldurchmesser des Kombinationsproduktes kleiner als 200 Mikrometer ist.

7. Verfahren zur Herstellung einer Pulvermischung nach einem der Ansprüche 1-6, dadurch gekennzeichnet, daß entweder ein mit dem Polymerisationsinitiator überzogenes Polymerisatpulver mit dem Röntgenkontrastmittel oder ein mit dem Polymerisationsinitiator überzogenes Röntgenkontrastmittel mit dem Polymerisatpulver vermischt wird.

8. Pulvermischung nach einem der Ansprüche 1-6, zur Verwendung in aushärtenden chirurgischen Massen auf der Basis von Polymethylmethacrylat oder seinen Mischpolmeren.

**Claims**

1. Powder mixture for surgical purposes, containing a methyl methacrylate homopolymer and/or copolymer powder, an X-ray contrast medium powder and a peroxide polymerisation initiator, characterised in that the peroxide polymerisation initiator is present as a coating on the polymer powder or on the X-ray contrast medium.

2. Powder mixture according to Claim 1, characterised in that the X-ray contrast medium is zirconium dioxide powder coated with benzoyl peroxide as the initiator.

3. Powder mixture according to Claim 1, characterised in that the polymer powder is coated with benzoyl peroxide.

4. Powder mixture according to Claim 1, characterised in that the initiator content is 1-50 % by weight, in particular 5-20 % by weight.

5. Powder mixture according to Claim 1, characterised in that the X-ray contrast medium content is up to 15 % by weight, in particular 5-10 % by weight.

6. Powder mixture according to Claim 1, characterised in that the maximum particle diameter of the combination product is less than 200 micrometres.

7. Process for the preparation of a powder mixture according to one of Claims 1-6, characterised in that either a polymer powder coated with the polymerisation initiator is mixed with the X-ray contrast medium or an X-ray contrast medium coated with the polymerisation initiator is mixed with the polymer powder.

8. Powder mixture according to one of Claims 1-6 for use in hardening surgical compositions based on polymethyl methacrylate or copolymers thereof.

**Revendications**

1. Mélange pulvérulent pour usage chirurgical, contenant de la poudre d'homopolymérisat et/ou de polymérisat mixte de méthacrylate de méthyle, de la poudre de moyen de contraste aux rayons X et un initiateur de polymérisation peroxydique, caractérisé en ce que l'initiateur de polymérisation peroxyde est présent en tant que revêtement de la poudre de polymérisat ou de moyen de contraste.

2. Mélange pulvérulent selon la revendication 1, caractérisé en ce que le moyen de contraste est de la poudre de dioxyde de zirconium, revêtue de peroxyde de benzoyle en tant qu'initiateur.

3. Mélange pulvérulent selon la revendication 1, caractérisé en ce que la poudre de polymérisat est revêtue de peroxyde de benzoyle.

4. Mélange pulvérulent selon la revendication 1, caractérisé en ce que la teneur en initiateur est comprise entre 1 et 50 % en poids, en particulier entre 5 et 20 % en poids.

5. Mélange pulvérulent selon la revendication 1, caractérisé en ce que la teneur en moyen de contraste s'élève jusqu'à 15 % en poids, en particulier 5 à 10 % en poids.

6. Mélange pulvérulent selon la revendication 1, caractérisé en ce que le diamètre maximal des particules du produit combiné est inférieur à 200 microns.

7. Procédé de préparation d'un mélange pulvérulent selon l'une des revendications 1-6, caractérisé en ce qu'on mélange : soit une poudre de polymérisat revêtue de l'initiateur de polymérisation avec le moyen de contraste, soit un moyen de contraste revêtu de l'initiateur de polymérisation avec la poudre de polymérisat.

8. Mélange pulvérulent selon l'une des revendications 1-6 pour utilisation dans les masses chirurgicales durcissantes sur la base du polyméthacrylate de méthyle ou de ses polymères mixtes.